(19) Europäisches Patentamt
European Patent Office
Office européen des brevets

(11) EP 1 286 646 B1

(12) **EUROPÄISCHE PATENTSCHRIFT**

(45) Veröffentlichungstag und Bekanntmachung des Hinweises auf die Patenterteilung:
**10.05.2006 Patentblatt 2006/19**

(21) Anmeldenummer: **01934014.0**

(22) Anmeldetag: **25.05.2001**

(51) Int Cl.:
***A61K 8/49*** *(2006.01)* ***A61Q 5/10*** *(2006.01)*

(86) Internationale Anmeldenummer:
**PCT/EP2001/006017**

(87) Internationale Veröffentlichungsnummer:
**WO 2001/093819 (13.12.2001 Gazette 2001/50)**

(54) **VERFAHREN ZUR OXIDATIVEN FÄRBUNG KERATINISCHER FASERN**
OXIDATION DYEING METHOD FOR KERATINOUS FIBERS
PROCEDE DE COLORATION PAR OXYDATION DE FIBRES DE KERATINE

(84) Benannte Vertragsstaaten:
**AT BE CH CY DE DK ES FI FR GB GR IE IT LI LU MC NL PT SE TR**

(30) Priorität: **06.06.2000 DE 10027975**

(43) Veröffentlichungstag der Anmeldung:
**05.03.2003 Patentblatt 2003/10**

(73) Patentinhaber: **Henkel Kommanditgesellschaft auf Aktien**
**40589 Düsseldorf (DE)**

(72) Erfinder:
- **NEUHAUS, Winifried**
**40625 Düsseldorf (DE)**
- **HOLLENBERG, Detlef**
**40699 Erkrath (DE)**

(56) Entgegenhaltungen:
**WO-A-99/66890**
**DE-A- 19 941 450**
**FR-A- 2 649 887**

EP 1 286 646 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft ein mehrstufiges Verfahren zur oxidativen Färbung keratinischer Fasern sowie die Verwendung von Mitteln, enthaltend Indolinderivate, zur Vorbehandlung keratinischer Fasern im Rahmen eines solchen Verfahrens zur Verbesserung der Echtheitseigenschaften der resultierenden Färbung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintreten kann.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Häufig sind aber in der Praxis insbesondere bei den Modenuancen im Rotbereich auch bei den oxidativen Färbungen unerwünschte Nuancenverschiebungen durch äußere Einflüsse wie beispielsweise Licht, Shampoonieren und Schweiß zu beobachten. Ferner treten teilweise Unterschiede im Aufzugsverhalten der Farbstoffe auf die unterschiedlich vorbehandelten Faserabschnitte auf; so können beispielsweise die frisch nachgewachsenen Haaransätze eine geringfügig andere Nuance erhalten als die bereits durch verschiedenste äußere Einflüsse veränderten Haarlängen. Die Fähigkeit der Färbemittel, die unterschiedlichen Haarbereiche gleichmäßig anzufärben wird als Egalisierungsvermögen bezeichnet. Zusammenfassend werden diese Eigenschaften als Echtheitseigenschaften der resultierenden Färbung bezeichnet.

Es besteht daher nach wie vor die Aufgabe, oxidative Färbungen hinsichtlich ihrer Echtheitseigenschaften zu verbessern. Bereits die Überwindung der Nachteile hinsichtlich einer dieser Eigenschaften stellt eine deutliche Verbesserung des Standes der Technik dar.

Es wurde nun überraschenderweise gefunden, daß diese Nachteile des Standes der Technik mit einem speziellen mehrstufigen Färbeverfahren überwunden werden können, bei dem die Fasern zunächst mit einem Mittel, enthaltend mindestens ein Indolinderivat, behandelt werden.

Ein erster Gegenstand der vorliegenden Erfindung ist daher ein Verfahren zur Färbung keratinischer Fasern, bei dem die Fasern

- in einem ersten Schritt mit einem Mittel (A), enthaltend mindestens ein Indolinderivat behandelt werden,
- gegebenenfalls das Mittel (A) in einem zweiten Schritt von den Fasern entfernt wird und
- in einem dritten Schritt die Fasern mit einem Mittel (B), enthaltend mindestens eine Entwickerkomponente, behandelt werden.

Unter keratinischen Fasern werden erfindungsgemäß Pelze, Wolle, Federn und insbesondere menschliche Haare verstanden.

In der internationalen Patentanmeldung WO-A1-93/09759 wird die Verwendung von speziellen Indolinderivaten als Farbstoffkomponente zusammen mit bekannten direktziehenden Farbstoffen oder mit Oxidationsfarbstoffvorprodukten vom Kuppler- und Entwicklertyp zur Erzeugung von Färbungen beschrieben. Dieser Schrift sind aber keinerlei Hinweise auf das erfindungsgemäße mehrstufige Färbeverfahren zu entnehmen.

FR-A-2 649 887 befaßt sich mit einem Verfahren zur Färbung von Keratinfasern, bei dem die Fasern zunächst mit einem Mittel, enthaltend ein Indolderivat und danach mit einem Mittel, enthaltend eine Entwicklerkomponente und eventual Wasserstoffperoxid behandelt werden. Das erste Mittel kann in einem Zwischenschritt von den Fasern entfernt werden.

Erfindungsgemäß bevorzugt sind Verfahren, bei denen das Mittel (A) mindestens ein Indolinderivat der Formel (Ia)

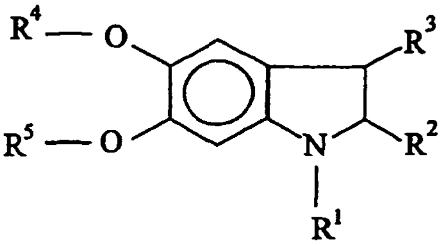

(Ia)

in der unabhängig voneinander

- $R^1$ steht für Wasserstoff, eine $C_1$-$C_4$-Alkylgruppe oder eine $C_1$-$C_4$-Hydroxy-alkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,

oder ein physiologisch verträgliches Salze dieser Verbindungen enthält

Beispiele für die als Substituenten in den erfindungsgemäßen Verbindungen genannten $C_1$-bis $C_4$-Alkylgruppen sind die Gruppen Methyl, Ethyl, Propyl, Isopropyl und Butyl. Ethyl und Methyl sind bevorzugte Alkylgruppen. Bevorzugte $C_1$-$C_4$-Hydroxyalkylgruppen sind die Gruppen Hydroxymethyl, 2-Hydroxyethyl, 3-Hydroxypropyl oder 4-Hydroxybutyl; 2-Hydroxyethyl ist eine besonders bevorzugte Hydroxyalkylgruppe. Da es sich bei allen erfindungsgemäßen Substanzen um Amino-Verbindungen handelt, lassen sich aus diesen in üblicher Weise die bekannten Säureadditionssalze herstellen. Beispiele für solche Salze sind die Hydrochloride, die Hydrobromide, die Sulfate, die Phosphate, die Acetate, die Propionate, die Citrate und die Lactate.

Besonders bevorzugte Verbindungen der Formel (Ia) sind ausgewählt aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und 5,6-Dihydroxyindolin-2-carbonsäure. 5,6-Dihydroxyindolin ist eine ganz besonders bevorzugte Komponente der Formel (I).

Weiterhin sind aber auch die Indolinderivate 6-Hydroxyindolin, 6-Aminoindolin und 4-Aminoindolin erfindungsgemäß bevorzugt.

In einer weiteren Ausführungsform der vorliegenden Erfindung kann das Mittel (A) mindestens ein Indolderivat und mindestens ein Indolinderivat enthalten.

Bevorzugt sind Indolderivate der Formel (Ib)

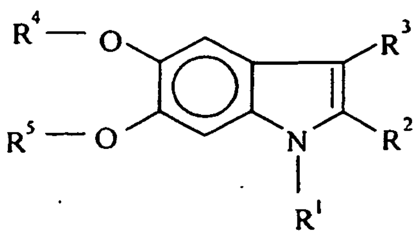

(Ib)

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der
- $R^6$ steht für eine $C_1$-$C_4$-Alkylgruppe, und
- $R^5$ steht für eine der unter $R^4$ genannten Gruppen,

sowie eines der physiologisch verträglichen Salze dieser Verbindungen.

Beispiele für die als Substituenten in den erfindungsgemäßen Verbindungen genannten $C_1$-bis $C_4$-Alkylgruppen sind die Gruppen Methyl, Ethyl, Propyl, Isopropyl und Butyl. Ethyl und Methyl sind bevorzugte Alkylgruppen. Bevorzugte $C_1$-$C_4$-Hydroxyalkylgruppen sind die Gruppen Hydroxymethyl, 2-Hydroxyethyl, 3-Hydroxypropyl oder 4-Hydroxybutyl; 2-Hydroxyethyl ist eine besonders bevorzugte Hydroxyalkylgruppe. Da es sich bei allen erfindungsgemäßen Substanzen um Amino-Verbindungen handelt, lassen sich aus diesen in üblicher Weise die bekannten Säureadditionssalze herstellen. Beispiele für solche Salze sind die Hydrochloride, die Hydrobromide, die Sulfate, die Phosphate, die Acetate, die Propionate, die Citrate und die Lactate.

Besonders bevorzugte Indolderivate der Formel (Ib) sind ausgewählt aus 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol und 5,6-Dihydroxyindol-2-carbonsäure. 5,6-Dihydroxyindol ist ein ganz besonders bevorzugtes Indolderivat der Formel (Ib).

Weiterhin haben sich auch die Indolderivate 6-Hydroxyindol, 6-Aminoindol und 4-Aminoindol als erfindungsgemäß geeignet erwiesen.

Das Mittel (B) enthält im Rahmen des erfindungsgemäßen Verfahrens mindestens eine Entwicklerkomponente.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (II)

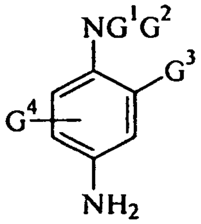

(II)

wobei

- $G^1$ steht für ein Wasserstoffatom, ein $C_1$- bis $C_4$-Alkylradikal, ein $C_1$- bis $C_4$-Monohydroxyalkylradikal, ein $C_2$- bis $C_4$-Polyhydroxyalkylradikal, ein ($C_1$- bis $C_4$)-Alkoxy-($C_1$- bis $C_4$)-alkylradikal, ein 4'-Aminophenylradikal oder ein $C_1$- bis $C_4$-Alkylradikal, das mit einer stickstoffhaltigen Gruppe, einem Phenyl- oder einem 4'-Aminophenylrest substituiert ist;
- $G^2$ steht für ein Wasserstoffatom, ein $C_1$- bis $C_4$-Alkylradikal, ein $C_1$- bis $C_4$-Monohydroxyalkylradikal, ein $C_2$- bis $C_4$-Polyhydroxyalkylradikal, ein ($C_1$- bis $C_4$)-Alkoxy-($C_1$- bis $C_4$)-alkylradikal oder ein $C_1$- bis $C_4$-Alkylradikal, das mit einer stickstoffhaltigen Gruppe substituiert ist;
- $G^3$ steht für ein Wasserstoffatom, ein Halogenatom, wie ein Chlor-, Brom, Jod-oder Fluoratom, ein $C_1$- bis $C_4$-Alkylradikal, ein $C_1$- bis $C_4$-Monohydroxyalkylradikal, ein $C_1$- bis $C_4$-Hydroxyalkoxyradikal, ein $C_1$- bis $C_4$-Acetylaminoalkoxyradikal, ein $C_1$- bis $C_4$- Mesylaminoalkoxyradikal oder ein $C_1$- bis $C_4$-Carbamoylaminoalkoxyradikal;
- $G^4$ steht für ein Wasserstoffatom, ein Halogenatom oder ein $C_1$- bis $C_4$-Alkylradikal oder
- wenn $G^3$ und $G^4$ in ortho-Stellung zueinander stehen, können sie gemeinsam eine verbrückende $\alpha,\omega$-Alkylendioxogruppe, wie beispielsweise einen Ethylendioxygruppe bilden.

Beispiele für die als Substituenten in den erfindungsgemäßen Verbindungen genannten, $C_1$- bis $C_4$-Alkylradikale sind die Gruppen Methyl, Ethyl, Propyl, Isopropyl und Butyl. Ethyl und Methyl sind bevorzugte Alkylradikale. Erfindungsgemäß bevorzugte $C_1$- bis $C_4$-Alkoxyradikale sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyalkylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, $C_1$- bis $C_4$-Monoalkylaminogruppen, $C_1$- bis $C_4$-Dialkylaminogruppen, $C_1$- bis $C_4$-Trialkyl-ammoniumgruppen, $C_1$- bis $C_4$-Monohydroxyalkylaminogruppen, Imidazolinium und Ammonium.

Besonders bevorzugte p-Phenylendiamine der Formel (II) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,-β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylamino-ethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (II) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (III) entsprechen, sowie ihre physiologisch verträglichen Salze:

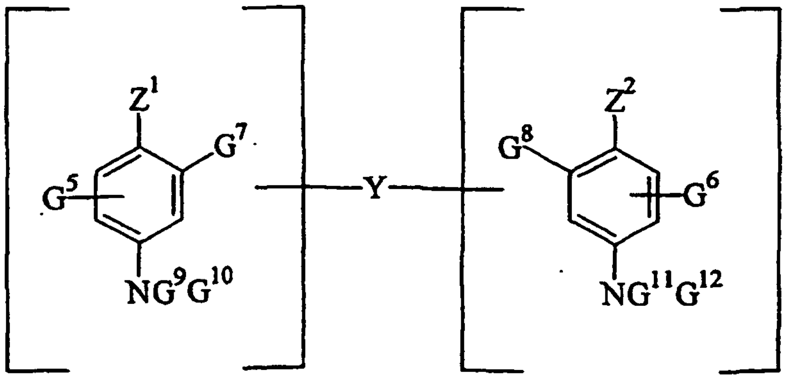

(III)

wobei:

- $Z^1$ und $Z^2$ stehen unabhängig voneinander für ein Hydroxyl- oder $NH_2$-Radikal, das gegebenenfalls durch ein $C_1$- bis $C_4$-Alkylradikal, durch ein $C_1$- bis $C_4$-Hydroxyalkylradikal und/oder durch eine Verbrückung Y substituiert ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder $C_1$- bis $C_8$-Alkoxyradikale substituiert sein kann,
- $G^5$ und $G^6$ stehen unabhängig voneinander für ein Wasserstoff- oder Halogenatom, ein $C_1$- bis $C_4$-Alkylradikal, ein $C_1$- bis $C_4$-Monohydroxyalkylradikal, ein $C_2$- bis $C_4$-Polyhydroxyalkylradikal, ein $C_1$- bis $C_4$-Aminoalkylradikal oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder ein $C_1$- bis $C_4$-Alkylradikal,
  mit der Maßgabe, daß die Verbindungen der Formel (III) nur eine Verbrückung Y pro Molekül enthalten.

Die in Formel (III) verwendeten Substituenten sind erfindungsgemäß analog zu den obigen Ausführungen definiert.

Bevorzugte zweikernige Entwicklerkomponenten der Formel (III) sind insbesondere: N,N'-bis-(β-Hydroxyethyl)-N,N'-bis-(4'-Aminophenyl)-1,3-diamino-propanol, N,N'-bis-(β-Hydroxyethyl)-N,N'-bis-(4'-ammophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N'-bis-(Ethyl)-N,N'-bis(4'-amino,3'-methylphenyl)-ethylendiamin, 1,8-bis-(2,5-Diaminophenoxy)-3,5-dioxaoktan, Bis-(2-hydroxy-5-aminophenyl)-methan, 1,4-Bis-(4-aminophenyl)-diaza-cycloheptan und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (III) sind N,N'-bis-(β-Hydroxyethyl)-N,N'-bis-(4'-aminophenyl)-1,3-diamino-propanol, Bis-(2-hydroxy-5-aminophenyl)-methan, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder

eines seiner physiologisch verträglichen Salze einzusetzen. Besonders bevorzugt sind p-Aminophenolderivate der Formel (IV)

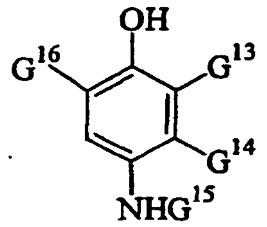

(IV)

wobei:

- $G^{13}$ steht für ein Wasserstoffatom, ein Halogenatom, ein $C_1$- bis $C_4$-Alkylradikal, ein $C_1$- bis $C_4$-Monohydroxyalkylradikal, ein ($C_1$- bis $C_4$)-Alkoxy-($C_1$- bis $C_4$)-alkylradikal, ein $C_1$- bis $C_4$-Aminoalkylradikal, ein Hydroxy-($C_1$- bis $C_4$)-alkylaminoradikal, ein $C_1$- bis $C_4$-Hydroxyalkoxyradikal, ein $C_1$- bis $C_4$-Hydroxyalkyl-($C_1$- bis $C_4$)-aminoalkylradikal oder ein (Di-$C_1$- bis $C_4$-Alkylamino)-($C_1$- bis $C_4$)-alkylradikal, und
- $G^{14}$ steht für ein Wasserstoff- oder Halogenatom, ein $C_1$- bis $C_4$-Alkylradikal, ein $C_1$- bis $C_4$-Monohydroxyalkylradikal, ein $C_2$- bis $C_4$-Polyhydroxyalkylradikal, ein ($C_1$- bis $C_4$)-Alkoxy-($C_1$- bis $C_4$)-alkylradikal, ein $C_1$- bis $C_4$-Aminoalkylradikal oder ein $C_1$- bis $C_4$-Cyanoalkylradikal,
- $G^{15}$ steht für Wasserstoff, ein $C_1$- bis $C_4$-Alkylradikal, ein $C_1$- bis $C_{4-}$Monohydroxyalkyltadikal, ein $C_2$- bis $C_4$-Polyhydroxyalkylradikal, ein Phenylradikal oder ein Benzylradikal, und
- $G^{16}$ steht für Wasserstoff oder ein Halogenatom.

Die in Formel (IV) verwendeten Substituenten sind erfindungsgemäß analog zu den obigen Ausführungen definiert.

Bevorzugte p-Aminophenole der Formel (IV) sind insbesondere p-Aminophenol, N-Methyl-p-Aminophenol, 4-Amino-3-methyl-phenol, 4-Amino-3-fluorphenol, 2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (IV) sind p-Aminophenol, 4-Amino-3-methylphenol, 4-Amino-2-aminomethylphenol und 4-Amino-2-((diethylamino)methyl)-phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamiao-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. 2,4,5,6-Tetraaminopyrimidin ist ein ganz besonders bevorzugtes Pyrimidinderivat.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol,

4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl) amino-1-methylpyrazol.

Bevorzugte Pyrazol-Pyrimidin-Derivate sind insbesondere die Derivate des Pyrazol-[1,5-a]-pyrimidin der folgenden Formel (V) und dessen tautomeren Formen, sofern ein tautomerisches Gleichgewicht besteht:

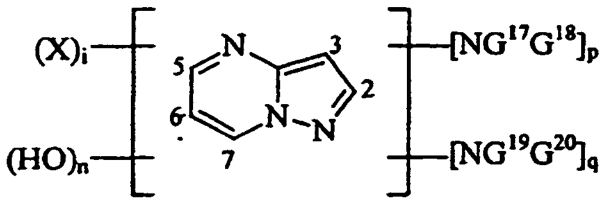

(V)

wobei:

- $G^{17}$, $G^{18}$, $G^{19}$ und $G^{20}$ unabhängig voneinander stehen für ein Wasserstoffatom, ein $C_1$- bis $C_4$-Alkylradikal, ein Aryl-Radikal, ein $C_1$- bis $C_4$-Hydroxyalkylradikal, ein $C_2$- bis $C_4$-Polyhydroxyalkylradikal ein ($C_1$- bis $C_4$)-Alkoxy-($C_1$- bis $C_4$)-alkylradikal, ein $C_1$- bis $C_4$-Aminoalkylradikal, das gegebenenfalls durch ein Acetyl-Ureid- oder Sulfonyl-Radikal geschützt sein kann, ein ($C_1$- bis $C_4$)-Alkylamino-($C_1$- bis $C_4$)-alkylradikal, ein Di-[($C_1$- bis $C_4$)-alkyl]-($C_1$-bis $C_4$)-aminoalkylradikal, wobei die Dialkyl-Radikale gegebenenfalls einen Kohlenstoffzyklus oder einen Heterozyklus mit 5 oder 6 Kettengliedern bilden, ein $C_1$- bis $C_4$-Hydroxyalkyl- oder ein Di-($C_1$- bis $C_4$)-[Hydroxyalkyl]-($C_1$- bis $C_4$)-aminoalkylradikal,
- die X-Radikale stehen unabhängig voneinander für ein Wasserstoffatom, ein $C_1$-bis $C_4$-Alkylradikal, ein Aryl-Radikal, ein $C_1$- bis $C_4$-Hydroxyalkyladikal, ein $C_2$- bis $C_4$-Polyhydroxyalkylradikal, ein $C_1$- bis $C_4$-Aminoalkylradikal, ein ($C_1$- bis $C_4$)-Alkylamino-($C_1$- bis $C_4$)-alkylradikal, ein Di-[($C_1$- bis $C_4$)alkyl]- ($C_1$- bis $C_4$)-aminoalkylradikal, wobei die Dialkyl-Radikale gegebenenfalls einen Kohlenstoffzyklus oder einen Heterozyklus mit 5 oder 6 Kettengliedern bilden, ein $C_1$- bis $C_4$-Hydroxyalkyl- oder ein Di-($C_1$- bis $C_4$-hydroxyalkyl)-aminoalkylradikal, ein Aminoradikal, ein $C_1$- bis $C_4$-Alkyl- oder Di-($C_1$- bis $C_4$-hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
  mit der Maßgabe, daß
- die Summe aus p + q ungleich 0 ist,
- wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
- wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (V) verwendeten Substituenten sind erfindungsgemäß analog zu den obigen Ausführungen definiert.

Wenn das Pyrazol-[1,5-a]-pyrimidin der obenstehenden Formel (V) eine Hydroxygruppe an einer der Positionen 2, 5 oder 7 des Ringsystems enthält, besteht ein tautomeres Gleichgewicht, das zum Beispiel im folgenden Schema dargestellt wird:

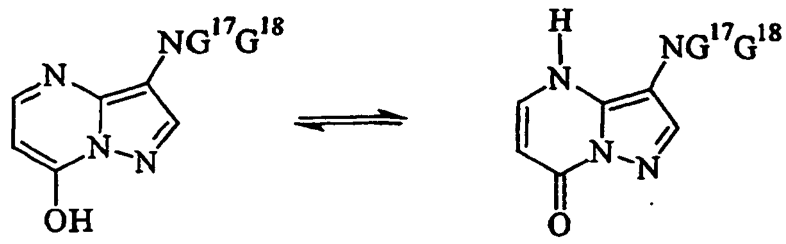

Unter den Pyrazol-[1,5-a]-pyrimidinen der obenstehenden Formel (V) kann man insbesondere nennen:

- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;

sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (V) können wie in der Literatur beschrieben durch Zyklisierung ausgehend von einem Aminopyrazol oder von Hydrazin hergestellt werden.

Weiterhin kann das Mittel (B) im Rahmen des erfindungsgemäßen Verfahrens mindestens eine Kupplerkomponente enthalten. Erfindungsgemäß bevorzugte Kupplerkomponenten sind

- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,

- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Di-hydroxy-2-methylpyrimidin, oder
- Methylendioxybenzolderivate wie beispielsweise 1-Hydroxy-3,4-methylen-dioxybenzol, 1-Amino-3,4-methylendioxybenzol und 1-(2'-Hydroxyethyl)-amino-3,4-methylendioxybenzol.

Besonders bevorzugt sind Kupplerkomponenten vom m-Aminophenoltyp. Weiterhin sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin erfindungsgemäß bevorzugte Kupplerkomponenten.

Die im Rahmen des erfindungsgemäßen Verfahrens eingesetzten Mittel enthalten die Wirkstoffe bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wäßrig-alkoholischen Lösungen sind im Sinne der vorliegenden Erfindung wäßrige Lösungen enthaltend 3 bis 70 Gew.% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Das Mittel (A) sollte bevorzugt einen pH-Wert im Bereich von 6 bis 12 aufweisen. Besonders bevorzugt ist die Anwendung eines Mittels (A), das schwach alkalisch eingestellt ist. Die Anwendungstemperaturen können in einem Bereich zwischen 15 und 40 °C liegen. Nach einer Einwirkungszeit von 0,5 bis 45 Minuten wird das Mittel gegebenenfalls von dem zu färbenden Haar entfernt. Dies kann sowohl durch gründliches Abstreichen als auch durch Ausspülen erfolgen. Gegebenenfalls kann anschließend eine Shampoonierung erfolgen. Das Nachwaschen mit einem Shampoo entfällt, wenn ein stark tensidhaltiger Träger verwendet wurde. In einer bevorzugten Ausführungsform werden die Fasern nach der Einwirkungszeit des Mittels (A) mit Wasser gespült.

Anschließend wird die eigentliche oxidative Färbung der Fasern durchgeführt. Diese kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorile und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen, wobei die Enzyme sowohl zur Erzeugung von oxidierenden PerVerbindungen eingesetzt werden als auch zur Verstärkung der Wirkung einer geringen Menge vorhandener Oxidationsmittel. So können die Enzyme (Enzymklasse I: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Das Mittel (B) wird zweckmäßigerweise unmittelbar vor der Anwendung durch Mischung der Zubereitung des Oxidationsmittels mit der Zubereitung, enthaltend die Oxidationsfarbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein zweiter Gegenstand der vorliegenden Erfindung ist die Verwendung eines Mittels, enthaltend mindestens ein Indolinderivat, zur Vorbehandlung keratinischer Fasern im Rahmen einer oxidativen Färbung zur Verbesserung der Echtheitseigenschaften der resultierenden Färbung.

Eine bevorzugte Ausführungsform dieses Gegenstandes ist die Verwendung eines Mittels, enthaltend mindestens ein Indolinderivat, zur Vorbehandlung keratinischer Fasern im Rahmen einer oxidativen Färbung zur Erhöhung der Waschechtheit.

Eine weitere bevorzugte Ausführungsform dieses Gegenstandes ist die Verwendung eines Mittels, enthaltend mindestens ein Indolinderivat, zur Vorbehandlung keratinischer Fasern im Rahmen einer oxidativen Färbung zur Erhöhung des Egalisiervermögens.

Ein dritter Gegenstand des vorliegenden Verfahrens ist ein Kit zur Färbung keratinischer Fasern, das eine erste Komponente, enthaltend mindestens ein Indolinderivat, und eine zweite Komponente, enthaltend mindestens eine Entwicklerkomponente, enthält

Ein vierter Gegenstand des vorliegenden Verfahrens ist ein Kit zur Färbung keratinischer Fasern, das eine erste Komponente, enthaltend mindestens ein Indolinderivat, eine zweite Komponente, enthaltend mindestens eine

Entwicklerkomponente, und als dritte Komponente eine Oxidationsmittelzubereitung enthält.

Die im Rahmen des erfindungsgemäßen Verfahrens eingesetzten Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R\text{-}O(CH_2\text{-}CH_2O)_x\text{-}SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8\text{-}C_{22}$-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}\text{-}C_{22}$-Fettsäuremono- und -diester von Anlagerungsprodukten von 1 bis 30 Mol Ethylenoxid an Glycerin,
- $C_8\text{-}C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel $R^1O\text{-}(Z)_X$. Diese Verbindungen sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Oetyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxy-ethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die im Rahmen des erfindungsgemäßen Verfahrens eingesetzten Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
  sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquatemium-22.

Als konditionierende Wirkstoffe weiterhin geeignet sind Silikonöle, insbesondere Dialkyl-und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Coming unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, $N_2O$, Dimethylether, $CO_2$ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

## Ansführungsbeispiele

Die folgenden Angaben sind, soweit nichts anderes vermerkt ist, in Gewichtsprozent.

**Zusammensetzung A**

| | |
|---|---|
| Stenol® 1618[1] | 6,7 |
| Kokoslorol®[2] | 2,0 |
| Eumulgin® B2[3] | 2,0 |
| Ammoniumsulfat | 1,0 |
| L(+)-Ascorbinsäure | 0,2 |
| 5,6-Dihydroxyindolin-hydrobromid | 0,5 |
| Ammoniak, 25%ig | ad pH 9 |
| Parfüm | q.s. |
| Wasser | ad 100 |

[1] $C_{16-18}$-Fettalkohol (INCI-Bezeichnung: Cetearyl alcohol) (Cognis)

[2] $C_{12-18}$-Fettalkohol (INCI-Bezeichnung: Coconut alcohol) (Cognis)

[3] Cetylstearylalkohol mit ca. 20 EO-Einheiten (INCI-Bezeichnung: Ceteareth-20) (Cognis)

**Zusammensetzung B**

| | |
|---|---|
| Hydrenol® D[4] | 5,5 |
| Kokoslorol® | 2,0 |
| Texapon® K14 S 70[5] | 2,8 |
| Lamesoft® PO 65[6] | 2,0 |
| Akypo® Soft 45 NV[7] | 10,0 |
| Eutanol® G[8] | 1,0 |
| Eumulgin® B1[9] | 0,5 |
| Eumulgin® B2 | 0,5 |
| Polymer[10] | 3,75 |
| Ammoniumsulfat | 0,7 |
| Natriumsulfit, wasserfrei | 0,5 |
| p-Toluylendiamin-sulfat | 0,3 |
| 1-(β-Hydroxyethylamino)-4-methyl-2-nitrobenzol | 0,1 |
| 4-Chlorresorcin | 0,1 |
| Turpinal® SL[11] | 0,2 |
| L(+)-Ascorbinsäure | 0,4 |
| 4-Amino-3-methylphenol | 0,9 |
| 2,7-Dihydroxynaphthalin | 0,2 |
| 5-Amino-2-methylphenol | 0,8 |
| 1,2-Propylenglykol | 2,5 |
| Ammoniak, 25%ig | ad pH 10 |
| Parfum | q.s. |
| Wasser | ad 100 |

[4] $C_{16-18}$-Fettalkohol (INCI- Bezeichnung: Cetearyl aleohol) (Cognis)

[5] Laurylmyristylethersulfat-Natrium-Salz (INCI-Bezeichnung: Sodium Myreth Sulfate, ca. 70% Aktivsubstanz) (Cognis)

[6] Alkylpolyglucosid-Ölsäuremonoglycerid-Gemisch (INCI-Bezeichnung: Coco-Glucoside, Glyceryl Oleate, ca. 70% Aktivsubstanz) (Cognis)

Tabelle fortgesetzt

[7] $C_{12-14}$-Fettalkohol- 4.5- EO- Essigsäure- Natrium- Salz (INCI-Bezeichnung: Sodium Laureth- 6 Carboxylate, ca. 21% Aktivsubstanz) (Chem- Y)

[8] 2-Octyldodecylalkohol (INCI-Bezeichnung: Octyldodecanol) (Cognis)

[9] Cetylstearylalkohol mit ca. 12 EO-Einheiten (INCI-Bezeichnung: Ceteareth-20) (Cognis)

[10] Copolymer aus Natriumacrylat und Acrylamidopropyltrimethylammoniumchlorid (INCI-Bezeichnung: Acrylamidopropyltrimonium Chloride/Acrylates Copolymer, ca. 20% Aktivsubstanz) (Stockhausen)

[11] 1-Hydroxyethan-1,1-diphosphonsäure (INCI-Bezeichnung: Etidronic Acid, ca. 60% Aktivsubstanz) (Cognis)

**Vorschädigung der Strähnen**

Die Versuche wurden an Egalisierungssträhnen (Kerling Nahuhaar, 80% meliert, 1,2g) durchgeführt. Hierzu wurden diese Strähnen in zwei Bereiche (oberer Teil und unterer Teil) aufgeteilt und jeweils unterschiedlich vorgeschädigt.

**Schädigung I (Oberer Teil der Strähne)**

Die Strähnen wurden mit dem Handelsprodukt Poly Blonde Ultra für 30min bei Raumtemperatur blondiert. Anschließend wurden die Strähnen gründlich gespült und getrocknet. Die Strähnen wurden nach dieser Vorschädigung 14 Tage bei Raumtemperatur gelagert, bevor die eigentlichen Versuche durchgeführt wurden.

**Schädigung II (Unterer Teil der Strähne)**

Die Strähnen wurden einer Dauerwellbehandlung mit dem Handelsprodukt Poly Lock Normal unterzogen. Dabei wurden die Strähnen zunächst 30min mit der Dauerwelllösung behandelt, gründlich gespült, mit einem Handtuch getrocknet und anschließend für 10min mit der Fixierlösung behandelt Nach Abspülen der Fixierlösung wurden die Strähnen mit einem Föhn getrocknet. Nach der Dauerwellbehandlung wurden die Strähnen mit dem Handelsprodukt Poly Blonde Ultra für 30min bei Raumtemperatur blondiert. Anschließend wurden die Strähnen gründlich gespült und erneut getrocknet. Die Behandlungsfolge Dauerwellbehandlung/Blondierung wurde an diesen Strähnen ein zweites Mal durchgeführt. Die Strähnen wurden nach dieser Vorschädigung 14 Tage bei Raumtemperatur gelagert bevor die eigentlichen Versuche durchgeführt wurden.

**Versuchsdurchführung**

Die unterschiedlich geschädigten Strähnen wurden jeweils mit 1,5g der Zusammensetzung A behandelt. Nach einer Einwirkungszeit von 5 Minuten wurde die Creme mit Wasser abgespült und auf das gut gespülte, handtuchtrockene Haar eine Mischung aus 0,75g der Zusammensetzung B und 0,75g einer wäßrigen 6%igen Wasserstoffperoxidlösung aufgetragen. Nach einer Einwirkungszeit von 30 Minuten wurden die Fasern erneut ausgewaschen.
Als Blindwert dienten analog vorgeschädigte Egalisierungssträhnen, die zunächst gut gespült und mit dem Handtuch getrocknet wurden. Anschließend wurde eine Mischung aus 0,75g der Zusammensetzung B und 0,75g einer wäßrigen 6%igen Wasserstoffperoxidlösung aufgetragen. Nach einer Einwirkungszeit von 30 Minuten wurden die Fasern erneut ausgewaschen.
Anschließend wurden alle Strähnen für 24h bei Raumtemperatur aufbewahrt. Danach wurden die Strähnen farbmetrisch vermessen. Zur Bestimmung der Waschechtheit wurden die Strähnen im Ultraschallbad in einer Texapon® NSO[12]-Lösung (10g Texapon® NSO / 1 Wasser) bei Raumtemperatur für l5min gewaschen und anschließend erneut farbmetrisch vermessen.

[12.] Natriumlaurylethersulfat (INCI-Bezeichnung: Sodium Laureth Sulfate, ca. 27% Aktivsubstanz) (Cognis)

1) Bestimmung des Egalisierungsvermögens vor und nach einmaligem Waschen

| Versuch | | L-Wert | a-Wert | b-Wert | ΔE |
|---|---|---|---|---|---|
| Blindwert vor dem Waschen | Schädigung I | 35,91 | 18,83 | 16,81 | 16,2 |
| | Schädigung II | 23,75 | 14,11 | 7,20 | |
| Vorbehandlung mit Zusammensetzung A vor dem Waschen | Schädigung I | 30,17 | 11,26 | 10,53 | 9,6 |
| | Schädigung II | 22,54 | 9,13 | 5,18 | |
| Blindwert nach dem Waschen | Schädigung I | 38,55 | 18,15 | 17,76 | 9,3 |
| | Schädigung II | 31,06 | 16,21 | 12,68 | |
| Vorbehandlung mit Zusammensetzung A nach dem Waschen | Schädigung I | 30,56 | 11,33 | 11,24 | 7,5 |
| | Schädigung II | 24,85 | 9,1 | 6,94 | |

2) Bestimmung der Waschechtheiten an den unterschiedlich vorgeschädigten Fasern

| Versuch | | L-Wert | a-Wert | b-Wert | ΔE-Wert |
|---|---|---|---|---|---|
| Blindwert Schädigung I | vor dem Waschen | 35,91 | 18,83 | 16,81 | 2,9 |
| | nach Waschen | 38,55 | 18,15 | 17,76 | |
| Vorbehandlung mit Zusammensetzung A Schädigung I | vor dem Waschen | 30,17 | 11,26 | 10,53 | 0,8 |
| | nach Waschen | 30,56 | 11,33 | 11,24 | |
| Blindwert Schädigung II | vor dem Waschen | 23,75 | 14,11 | 7,20 | 9,4 |
| | nach dem Waschen | 31,06 | 16,21 | 12,68 | |
| Vorbehandlung mit Zusammensetzung A Schädigung II | vor dem Waschen | 22,54 | 9,13 | 5,18 | 2,9 |
| | nach dem Waschen | 24,85 | 9,1 | 6,94 | |

Die ΔE-Werte des CIELAB-Farbsystems wurden gemäß Gleichung (I) ermittelt:

$$\Delta E = \sqrt{(\Delta L)^2 + (\Delta A)^2 + (\Delta B)^2} \qquad (I)$$

Den ΔE-Werten ist deutlich zu entnehmen, daß die mit 5,6-Dihydroxyindolin vorbehandelten Strähnen ein besseres Egalisiervermögen (kleinerer ΔE-Wert für den Vergleich Schädigung I / Schädigung II) und eine deutliche verbesserte Waschechtheit (kleinerer ΔE-Wert für den Vergleich vor dem Waschen/nach dem Waschen) als die mit dem herkömmlichen Verfahren behandelten Strähnen aufweisen.

## Patentansprüche

**1.** Verfahren zur Färbung keratinischer Fasern, **dadurch gekennzeichnet, dass** die Fasern

- in einem ersten Schritt mit einem Mittel (A), enthaltend mindestens ein Indolinderivat behandelt werden,
- gegebenenfalls das Mittel (A) in einem zweiten Schritt von den Fasern entfernt wird
- in einem dritten Schritt die Fasern mit einem Mittel (B), enthaltend mindestens eine Entwicklerkomponente, behandelt werden

**2.** Verfahren nach Anspruch 1, **dadurch gekennzeichnet, dass** das Mittel (A) mindestens ein Indolinderivat der Formel (Ia)

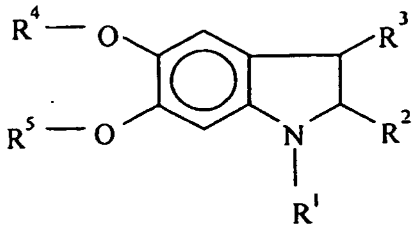

(Ia)

in der unabhängig voneinander
$R^1$ steht für Wasserstoff, eine $C_1$-$C_4$-Alkylgruppe oder eine $C_1$-$C_4$-Hydroxy-alkylgruppe,
$R^2$ steht für Wasserstoff oder eine -COOH-Gruppe, wobei die -COOH-Gruppe auch als Salz mit einem physiologisch verträglichen Kation vorliegen kann,
$R^3$ steht für Wasserstoff oder eine $C_1$-$C_4$-Alkylgruppe,
$R^4$ steht für Wasserstoff, eine $C_1$-$C_4$-Alkylgruppe oder eine Gruppe -CO-$R^6$, in der
$R^6$ steht für eine $C_1$-$C_4$-Alkylgruppe, und
$R^5$ steht für eine der unter $R^4$ genannten Gruppen,
oder ein physiologisch verträgliches Salz dieser Verbindungen enthält.

3. Verfahren nach Anspruch 2, **dadurch gekennzeichnet, dass** die Verbindung der Formel (Ia) ausgewählt ist aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und 5,6-Dihydroxyindolin-2-carbonsäure.

4. Verfahren nach Anspruch 3, **dadurch gekennzeichnet, dass** die Verbindung der Formel (Ia) 5,6-Dihydroxyindolin ist.

5. Verfahren nach einem der Ansprüche 1 bis 4, **dadurch gekennzeichnet, dass** das Mittel (B) als Entwicklerkomponente mindestens ein Pyrimidinderivat enthält.

6. Verfahren nach Anspruch 5, **dadurch gekennzeichnet, dass** das Pyrimidinderivat 2,4,5,6-Tetraaminopyrimidin ist.

7. Verfahren nach einem der Ansprüche 1 bis 6, **dadurch gekennzeichnet, dass** das Mittel (B) als Entwicklerkomponente mindestens ein p-Aminophenolderivat enthält.

8. Verfahren nach Anspruch 7, **dadurch gekennzeichnet, dass** das p-Aminophenolverivat ausgewählt ist aus p-Aminophenol, N-Methyl-p-Aminophenol, 4-Amino-3-methylphenol, 4-Amino-3-fluorphenol,2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihren physiologisch verträglichen Salzen.

9. Verfahren nach einem der Ansprüche 1 bis 8, **dadurch gekennzeichnet, dass** das Mittel (B) weiterhin mindestens eine Kupplerkomponente enthält.

10. Verfahren nach Anspruch 9, **dadurch gekennzeichnet, dass** die Kupplerkomponente ein m-Aminophenolderviat ist.

11. Verfahren nach Anspruch 10, **dadurch gekennzeichnet, dass** das m-Aminophenolderivat ausgewählt ist aus 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylpheno und 2,4-Dichlor-3-aminophenol.

12. Verwendung eines Mittels, enthaltend mindestens ein Indolinderivat, zur Vorbehandlung keratinischer Fasern im Rahmen einer oxidativen Färbung zur Verbesserung der Echtheitseigenschaften der resultierenden Färbung.

13. Verwendung nach Anspruch 12 zur Erhöhung der Waschechtheit der resultierenden Färbung.

**14.** Verwendung nach Anspruch 12 zur Erhöhung des Egalisiervermögens.

**15.** Kit zur Färbung keratinischer Fasern, **dadurch gekennzeichnet, dass** es eine erste Komponente, enthaltend mindestens ein Indolinderivat, und eine zweite Komponente, enthaltend mindestens eine Entwicklerkomponente, enthält.

**16.** Kit zur Färbung keratinischer Fasern, **dadurch gekennzeichnet, dass** es eine erste Komponente, enthaltend mindestens ein Indolinderivat, eine zweite Komponente, enthaltend mindestens eine Entwicklerkomponente, und als dritte Komponente eine Oxidationsmittelzubereitung enthält.

## Claims

**1.** Method of dyeing keratinous fibres, **characterized in that** the fibres

- are treated in a first step with an agent (A) comprising at least one indoline derivative,
- optionally the agent (A) is removed from the fibres in a second step and
- in a third step the fibres are treated with an agent (B) comprising at least one developer component.

**2.** Method according to Claim 1, **characterized in that** the agent (A) comprises at least one indoline derivative of the formula (Ia)

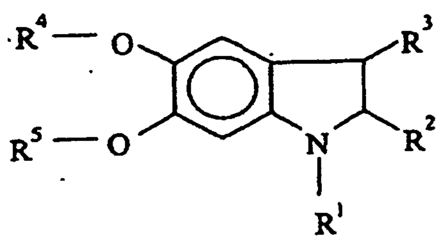

(Ia)

in which, independently of one another,
$R^1$ is hydrogen, a $C_1$-$C_4$-alkyl group or a $C_1$-$C_4$-hydroxyalkyl group,
$R^2$ is hydrogen or a -COOH group, where the -COOH group may also be present in the form of a salt with a physiologically compatible cation,
$R^3$ is hydrogen or a $C_1$-$C_4$-alkyl group,
$R^4$ is hydrogen, a $C_1$-$C_4$-alkyl group or a group -CO-$R^6$, in which
$R^6$ is a $C_1$-$C_4$-alkyl group, and
$R^5$ is one of the groups specified under $R^4$,
or a physiologically compatible salt of these compounds.

**3.** Method according to Claim 2, **characterized in that** the compound of the formula (Ia) is chosen from 5,6-dihydroxyindoline, N-methyl-5,6-dihydroxyindoline, N-ethyl-5,6-dihydroxyindoline, N-propyl-5,6-dihydroxyindoline, N-butyl-5,6-dihydroxyindoline and 5,6-dihydroxyindoline-2-carboxylic acid.

**4.** Method according to Claim 3, **characterized in that** the compound of the formula (Ia) is 5,6-dihydroxyindoline.

**5.** Method according to one of Claims 1 to 4, **characterized in that** the agent (B) comprises at least one pyrimidine derivative as developer component.

**6.** Method according to Claim 5, **characterized in that** the pyrimidine derivative is 2,4,5,6-tetraaminopyrimidine.

**7.** Method according to one of Claims 1 to 6, **characterized in that** the agent (B) comprises at least one p-aminophenol derivative as developer component.

**8.** Method according to Claim 7, **characterized in that** the p-aminophenol derivative is chosen from p-aminophenol,

N-methyl-p-aminophenol, 4-amino-3-methylphenol, 4-amino-3-fluorophenol, 2-hydroxymethylamino-4-aminophenol, 4-amino-3-hydroxymethylphenol, 4-amino-2-(2-hydroxyethoxy)phenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-fluorophenol, 4-amino-2-chlorophenol, 2,6-dichloro-4-aminophenol, 4-amino-2-((diethylamino)methyl)phenol, and their physiologically compatible salts.

**9.** Method according to one of Claims 1 to 8, **characterized in that** the agent (B) further comprises at least one coupler component.

**10.** Method according to Claim 9, **characterized in that** the coupler component is an m-aminophenol derivative.

**11.** Method according to Claim 10, **characterized in that** the m-aminophenol derivative is chosen from 5-amino-2-methylphenol, 3-amino-2-chloro-6-methylphenol, 2-hydroxy-4-aminophenoxyethanol, 2,6-dimethyl-3-aminophenol, 3-trifluoroacetylamino-2-chloro-6-methylphenol, 5-amino-4-chloro-2-methylphenol, 5-amino-4-methoxy-2-methylphenol, 5-(2'-hydroxyethyl)amino-2-methylphenol, 3-(diethylamino)phenol, N-cyclopentyl-3-aminophenol, 1,3-dihydroxy-5-(methylamino)benzene, 3-(ethylamino)-4-methylphenol and 2,4-dichloro-3-aminophenol.

**12.** Use of an agent comprising at least one indoline derivative for the pretreatment of keratinous fibres in the course of an oxidation dyeing for improving the fastness properties of the resulting colour.

**13.** Use according to Claim 12 for increasing the wash fastness of the resulting colour.

**14.** Use according to Claim 12 for increasing the equalizing power.

**15.** Kit for dyeing keratinous fibres, **characterized in that** it comprises a first component comprising at least one indoline derivative, and a second component comprising at least one developer component.

**16.** Kit for dyeing keratinous fibres, **characterized in that** it comprises a first component comprising at least one indoline derivative, a second component comprising at least one developer component, and, as third component, an oxidizing agent preparation.

## Revendications

**1.** Procédé pour la teinture de fibres kératiniques, **caractérisé en ce que** les fibres :

- dans une première étape, sont traitées avec un agent (A) contenant au moins un dérivé d'indoline ;
- éventuellement, l'agent (A), dans une deuxième étape, est éliminé des fibres ; et
- dans une troisième étape, les fibres sont traitées avec un agent (B) contenant au moins un composant faisant office de développeur.

**2.** Procédé selon la revendication 1, **caractérisé en ce que** l'agent (A) contient au moins un dérivé d'indoline répondant à la formule (Ia)

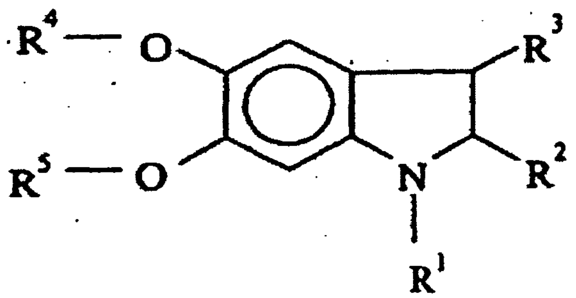

(Ia)

dans laquelle, indépendamment l'une de l'autre
$R^1$ représente un atome d'hydrogène, un groupe alkyle en $C_1$-$C_4$ ou un groupe hydroxyalkyle en $C_1$-$C_4$ ;

$R^2$ représente un atome d'hydrogène ou un groupe -COOH, le groupe -COOH pouvant également être présent sous la forme d'un sel avec un cation physiologiquement acceptable ;
$R^3$ représente un atome d'hydrogène ou un groupe alkyle en $C_1$-$C_4$ ;
$R^4$ représente un atome d'hydrogène, un groupe alkyle en $C_1$-$C_4$ ou un groupe -CO-$R^6$ dans lequel $R^6$ représente un groupe alkyle en $C_1$-$C_4$ ; et
$R^5$ représente un des groupes mentionnés sous $R^4$ ;
ou un sel physiologiquement acceptable de ces composés.

3. Procédé selon la revendication 2, **caractérisé en ce que** le composé répondant à la formule (Ia) est choisi parmi le groupe comprenant la 5,6-dihydroxyindoline, la N-méthyl-5,6-dihydroxyindoline, la N-éthyl-5,6-dihydroxyindoline, la N-propyl-5,6-dihydroxyindoline, la N-butyl-5,6-dihydroxyindoline et l'acide 5,6-dihydroxyindoline-2-carboxylique.

4. Procédé selon la revendication 3, **caractérisé en ce que** le composé répondant à la formule (Ia) est la 5,6-dihydroxyindoline.

5. Procédé selon l'une quelconque des revendications 1 à 4, **caractérisé en ce que** l'agent (B) contient, à titre de composant faisant office de développeur, au moins un dérivé de pyrimidine.

6. Procédé selon la revendication 5, **caractérisé en ce que** le dérivé de pyrimidine est la 2,4,5,6-tétraaminopyrimidine.

7. Procédé selon l'une quelconque des revendications 1 à 6, **caractérisé en ce que** l'agent (B) contient, à titre de composant faisant office de développeur, au moins un dérivé de p-aminophénol.

8. Procédé selon la revendication 7, **caractérisé en ce que** le dérivé de p-aminophénol est choisi parmi le groupe constitué par le p-aminophénol, le N-méthyl-p-aminophénol, le 4-amino-3-méthylphénol, le 4-amino-3-fluorophénol, le 2-hydroxyméthylamino-4-aminophénol, le 4-amino-3-hydroxyméthylphénol, le 4-amino-2-(2-hydroxyéthoxy)phénol, le 4-amino-2-méthylphénol, le 4-amino-2-hydroxyméthylphénol, le 4-amino-2-méthoxyméthylphénol, le 4-amino-2-aminométhylphénol, le 4-amino-2-(β-hydroxyéthyl-aminométhyl)phénol, le 4-amino-2-fluorophénol, le 4-amino-2-chlorophénol, le 2,6-dichloro-4-aminophénol, le 4-amino-2-((diéthylamino)méthyl)phénol, ainsi que leurs sels physiologiquement acceptables.

9. Procédé selon l'une quelconque des revendications 1 à 8, **caractérisé en ce que** l'agent (B) contient en outre au moins un composant faisant office de coupleur.

10. Procédé selon la revendication 9, **caractérisé en ce que** le composant faisant office de coupleur est un dérivé de m-aminophénol.

11. Procédé selon la revendication 10, **caractérisé en ce que** le dérivé de m-aminophénol est choisi parmi le groupe constitué par le 5-amino-2-méthylphénol, le 3-amino-2-chloro-6-méthylphénol, le 2-hydroxy-4-aminophénoxyéthanol, le 2,6-diméthyl-3-aminophénol, le 3-trifluoro-acétylamino-2-chloro-6-méthylphénol, le 5-amino-4-chloro-2-méthylphénol, le 5-amino-4-méthoxy-2-méthylphénol, le 5-(2'-hydroxyéthyl)amino-2-méthylphénol, le 3-(diéthylamino)phénol, le N-cyclopentyl-3-aminophénol, le 1,3-dihydroxy-5-(méthylamino)benzène, le 3-(éthylamino)-4-méthylphénol et le 2,4-dichloro-3-aminophénol.

12. Utilisation d'un agent, contenant au moins un dérivé d'indoline pour le prétraitement de fibres kératiniques dans le cadre d'une teinture par oxydation pour améliorer les propriétés de solidité de la teinture résultante.

13. Utilisation selon la revendication 12, pour augmenter la solidité au lavage de la teinture résultante.

14. Utilisation selon la revendication 12, pour augmenter le pouvoir d'égalisation.

15. Nécessaire pour la teinture de fibres kératiniques, **caractérisé en ce qu'**il contient un premier composant, contenant au moins un dérivé d'indoline et un deuxième composant contenant au moins un composant faisant office de développeur.

16. Nécessaire pour la teinture de fibres kératiniques, **caractérisé en ce qu'**il contient un premier composant, contenant au moins un dérivé d'indoline, un deuxième composant contenant au moins un composant faisant office de développeur et, à titre de troisième composant, une préparation d'agent d'oxydation.